Europäisches Patentamt

European Patent Office

Office européen des brevets

(19)

(11) Veröffentlichungsnummer: **0 198 322 B1**

## (12) EUROPÄISCHE PATENTSCHRIFT

(45) Veröffentlichungstag der Patentschrift: **10.04.91**

(51) Int. Cl.⁵: **C12Q 1/56**, C12Q 1/37

(21) Anmeldenummer: **86104476.6**

(22) Anmeldetag: **02.04.86**

(54) **Verfahren zur Bestimmung von Plasminogenaktivatoren (PA).**

(30) Priorität: **11.04.85 DE 3512909**

(43) Veröffentlichungstag der Anmeldung:
**22.10.86 Patentblatt 86/43**

(45) Bekanntmachung des Hinweises auf die
Patenterteilung:
**10.04.91 Patentblatt 91/15**

(84) Benannte Vertragsstaaten:
**AT BE CH DE FR GB IT LI LU NL SE**

(56) Entgegenhaltungen:
**EP-A- 0 094 720**
**WO-A-82/01377**

(73) Patentinhaber: **BEHRINGWERKE Aktiengesellschaft**
**Postfach 1140**
**W-3550 Marburg 1(DE)**

(72) Erfinder: **Pâques, Eric Paul, Dr.**
**Schmiedeacker 18**
**W-3550 Marburg(DE)**

(74) Vertreter: **Meyer-Dulheuer, Karl-Hermann, Dr.
et al**
**HOECHST Aktiengesellschaft Postfach 3540**
**W-6200 Wiesbaden(DE)**

**Beschreibung**

Die Erfindung betrifft ein Verfahren zur Bestimmung von Plasminogenaktivatoren (PA). Unter dem Begriff Plasminogenaktivator soll Urokinase (UK) und Gewebe-Plasminogenaktivator (t-PA) verstanden werden.

Plasminogen wird durch Plasminogenaktivatoren in Plasmin umgewandelt. Zu den Katalysatoren dieser Reaktion zählen Streptokinase, Urokinase und t-PA. Der Nachweis von PA in Körperflüssigkeitsproben von Patienten ist insbesondere für die Früherkennung von thrombose-gefährdeten Patienten von Bedeutung. Für die PA-Aktivitätsmessung sind Bestimmungsmethoden bekannt. Bedingt durch die geringe PA-Aktivität in Körperflüssigkeiten erfordern diese jedoch sehr lange Inkubationszeiten. Zur Beschleunigung kann für t-PA die Aktivität durch Zugabe von Fibrin oder Fibrin-Spaltprodukten stimuliert werden (Biochim.Biophys. Acta (1982) 704, 461-469). In EP 0 094 720 ist eine Methode für die Bestimmung von t-PA in Anwesenheit von Fibrin beschrieben. Die Herstellung von Fibrin oder Fibrin-Spalt-Produkten ist aufwendig, und die Anwendung von Fibrin oder Fibrin-Spaltprodukten ist auch durch die geringe Löslichkeit dieser Komponenten begrenzt.

Der vorliegenden Erfindung liegt die Aufgabe zugrunde, eine empfindlichere, schnellere und einfachere Nachweismethode als die bereits bekannte für PA zu entwickeln.

Es wurde überraschenderweise gefunden, daß die Aktivität von PA in Anwesenheit von Polyschwefelsäureester von Sacchariden erheblich gesteigert ist.

Es wurde weiterhin überraschenderweise gefunden, daß Polyschwefelsäureester von Sacchariden oder sulfatierten Zuckern den Stimulierungseffekt von Fibrin oder von Fibrin-Spaltprodukten auf die t-PA Aktivität in einem Testsystem, enthaltend t-PA und Plasminogen, ersetzen oder ergänzen können.

Gegenstand der Erfindung ist ein Verfahren zur Bestimmung eines Plasminogenaktivator (PA) in einer Körperflüssigkeit, dadurch gekennzeichnet, daß dieser PA mit Plasminogen und einem Polyschwefelsäureester eines Saccharids oder sulfatierten Zucker oder Zellmembranbestandteilen und gegebenenfalls mit Fibrin oder einem Fibrin-Spaltprodukt inkubiert und die Umsetzung von Plasminogen zu Plasmin bestimmt wird.

Eine solche Körperflüssigkeit ist beispielsweise Plasma.

Vorzugsweise wird zur Bestimmung der Umsetzung von Plasminogen zu Plasmin ein chromogenes Substrat verwendet.

Als Polyschwefelsäureester von Sacchariden oder sulfatierte Zucker können insbesondere Heparin, Heparan-Sulfat, Pentosansulfat, Dextransulfat, Keratansulfat, Chondroitinsulfat, Dermatansulfat oder Arteparon[R] Mucopolysaccharidpolyschwefelsäureester angewendet werden.

Vorzugsweise werden die Polyschwefelsäureester in einer Konzentration von $10^{-8}$ bis 1 mg/ml Testansatz eingesetzt. Vorzugsweise wird Heparin in einer Konzentration von $10^{-5}$ bis 1 mg/ml Testansatz eingesetzt.

In einer besonders bevorzugten Ausführungsform kann man für die Bestimmung der PA-Aktivität so verfahren, daß man 0,1 ml einer PA-haltigen Probe mit 0,1 ml einer Plasminogen-Lösung, enthaltend 0,1 bis 10, vorzugsweise 0,5-2 CTA/ml (Comittee of Thrombolytic Agents), 0,1 ml einer Lösung enthaltend $10^{-7}$ bis 10 mg/ml, vorzugsweise $10^{-5}$ bis 10 mg/ml, eines Polyschwefelsäureesters eines Saccharids oder sulfatierten Zuckers, vorzugsweise Heparin in einer Konzentration von $10^{-5}$ bis 10 mg/ml, vorzugsweise $10^{-4}$ bis 10 mg/ml, 0,7 ml eines Puffers, vorzugsweise 0,1 mol/l Tris-HCl, pH 6,5-8,5, gegebenenfalls 0,1 ml/100 ml Tween[R] 80 enthaltend, und ein plasminspezifisches chromogenes Substrat beispielsweise HD-Phe-Tyr-Arg-ANBS-ethylester, HD-Phe-Tyr-Lys-ANBS-neopentylamid oder HD-Val-Leu-Lys-pNA, bei einer Temperatur von 10-40° C, vorzugsweise 30-40° C inkubiert und die Menge des freigesetzten Chromophors bestimmt.

Es wurde auch überraschenderweise gefunden, daß auch Zellmembranbestandteile die PA-Aktivität stimulieren können.

In einer anderen besonders bevorzugten Ausführungsform kann man für die Bestimmung der PA-Aktivität so verfahren, daß man 0,1 ml einer PA-haltigen Probe, beispielsweise Plasma, mit 0,1 ml einer Plasminogenlösung, enthaltend 0,1 bis 10 CTA/ml, vorzugsweise 0,5-2 CTA/ml, 0,1 ml einer Lösung enthaltend lysierte Zellen, vorzugsweise Thrombozyten, vorzugsweise in einer Konzentration von 25.000 bis 2.500.000 Zellen/ml, oder Staphylokokken, vorzugsweise in einer Konzentration von $10^{-4}$ bis $10^{-1}$ g/ml, 0,7 ml eines Puffers, vorzugsweise 0,1 mol/l Tris-HCl, pH 6,5-8,5, gegebenenfalls 0,1 ml/100 ml Tween[R] 80 enthaltend, und ein plasmin-spezifisches chromogenes Substrat, beispielsweise HD-Phe-Tyr-Arg-ANBS-ethylester, HD-Phe-Tyr-Lys-ANBS-neopentylamid oder HD-Val-Leu-Lys-pNA, bei einer Temperatur von 10-40° C, vorzugsweise 30-40° C inkubiert und die Menge des freigesetzten Chromophors bestimmt.

Zur Auswertung kann eine Eichkurve verwendet werden. Dem Reaktionsansatz kann bei der t-PA-Aktivitätsbestimmung Fibrin oder ein Fibrin-Spaltprodukt, je nach Anteil an plasminogenaktivator-stimulierenden Peptiden, in einer Konzentration von 0,001 bis 1 mg/ml im Testansatz zugesetzt werden.

Die beschriebene Bestimmungsmethode für PA zeichnet sich besonders dadurch aus, daß PA stimuliert, damit die Aktivierung von Plasminogen beschleunigt und folglich die Empfindlichkeit gesteigert ist.

Gegenstand der Erfindung ist weiterhin ein Mittel zur Bestimmung eines Plasminogenaktivators mindestens enthaltend 0,01 bis 1 CTA/ml Plasminogen und $10^{-4}$ bis 1 mg/ml Heparin sowie gegebenenfalls 0,01 bis 0,1 ml/100 ml Polyoxyethylensorbitan-monooleat und gegebenenfalls Fibrinspaltprodukte, ein solches Mittel mindestens enthaltend 0,01 bis 1 CTA/ml Plasminogen und $2,5 \times 10^3$ bis $2,5 \times 10^5$ Thrombozyten/ml sowie gegebenenfalls 0,01 bis 0,1 ml/100 ml Polyoxyethylensorbitan-monooleat und gegebenenfalls Fibrinspaltprodukte sowie ein solches Mittel mindestens enthaltend 0,01 bis 1 CTA/ml Plasminogen und $10^{-1}$ bis 10 mg Staphylokokken/ml sowie gegebenenfalls 0,01 bis 0,1/100 ml Polyoxyethylensorbitan-monooleat und gegebenenfalls Fibrinspaltprodukte.

Die Erfindung soll durch die nachstehenden Beispiele näher erläutert werden.

Beispiel 1

Der Testansatz bestand aus 0,1 ml t-PA-haltiger Probe, 0,1 ml Plasminogenlösung (1 CTA/ml), 0,7 ml eines Puffers, enthaltend 0,1 mol/l Tris-HCl, pH 7,5, 0,1 ml/100 ml Tween$^R$ 80 und dem Plasmin-Substrat HD-Phe-Leu-Lys-PNA (Kabi, Schweden) und zusätzlich 0,1 ml einer Heparinlösung mit verschiedenen Konzentrationen. Der Ansatz wurde 20 min bei 37° C inkubiert, anschließend die Farbstoffentwicklung gestoppt und die $OD_{405nm}$ gemessen.

Tabelle 1 zeigt das Ergebnis.

**Tabelle 1**

| Zugesetzte Heparin-Konzentrationen (USP/ml Testansatz) | $OD_{405nm}$ | Stimulierungsfaktor |
|---|---|---|
| 0 | 0,082 | 1,00 |
| 0,005 | 0,103 | 1,26 |
| 0,05 | 0,406 | 4,95 |
| 0,5 | 0,941 | 11,48 |
| 5 | 0,594 | 7,23 |
| 50 | 0,097 | 1,18 |

USP = United States Pharmacopea

Beispiel 2

Wie in Beispiel 1, wobei 0,1 ml einer thrombozytenhaltigen Lösung mit verschiedenen Konzentrationen

3

anstelle der Heparinlösung, zugesetzt wurden.
Tabelle 2 zeigt das Ergebnis.

## Tabelle 2

| Zugesetzte Thrombozyten-Konzentrationen (Zellen/ml Testansatz) | $OD_{405nm}$ | Stimulierungsfaktor |
|---|---|---|
| 0 | 0,082 | 1,00 |
| 2,5 | 0,109 | 1,33 |
| 25 | 0,116 | 1,42 |
| 250 | 0,190 | 2,32 |
| 2500 | 0,938 | 11,44 |
| 25000 | ⟩ 3,000 | ⟩ 36,59 |
| 250000 | 2,126 | 25,93 |

Beispiel 3

Wie in Beispiel 1, wobei 0,1 ml einer Lösung lysierter Staphylokokken mit verschiedenen Konzentrationen anstelle der Heparinlösung, zugesetzt wurden.
Tabelle 3 zeigt das Ergebnis.

4

## Tabelle 3

| Zugesetzte Staphylo-kokken-Konzentrationen (mg Staphylokokken/ml Testansatz) | OD$_{405nm}$ | Stimulierungsfaktor |
|---|---|---|
| 0 | 0,082 | 1,00 |
| $10^{-4}$ | 0,116 | 1,42 |
| $10^{-3}$ | 0,200 | 2,44 |
| $10^{-2}$ | 0,140 | 1,71 |
| $10^{-1}$ | 1,468 | 17,90 |
| 1 | ⟩ 3,000 | ⟩ 36,59 |
| 10 | 2,460 | 30 |

Beispiel 4

Wie in Beispiel 1 mit einer UK-haltigen Lösung
Tabelle 4 zeigt das Ergebnis.

## Tabelle 4

| Zugesetzte Heparin-Konzentrationen (USP/ml Testansatz) | $OD_{405nm}$ | Stimulierungsfaktor |
|---|---|---|
| 0 | 0,151 | 1,00 |
| 0,005 | 0,420 | 2,78 |
| 0,05 | 0,676 | 4,48 |
| 0,5 | 0,971 | 6,43 |
| 5 | 0,978 | 6,48 |
| 50 | 0,584 | 3,87 |

**USP = United States Pharmacopea**

Beispiel 5

Wie in Beispiel 2 mit einer UK-haltigen Lösung
Tabelle 5 zeigt das Ergebnis.

Tabelle 5

| Zugesetzte Thrombozyten-Konzentrationen (Zellen/ml Testansatz) | $OD_{405nm}$ | Stimulierungsfaktor |
|---|---|---|
| 0 | 0,151 | 1,00 |
| 2,5 | 0,224 | 1,48 |
| 25 | 0,227 | 1,50 |
| 250 | 0,269 | 1,78 |
| 2500 | 0,459 | 3.04 |
| 25000 | 3,750 | 24,83 |
| 250000 | 0,385 | 2,55 |

Beispiel 6

Wie in Beispiel 3 mit einer UK-haltigen Lösung
Tabelle 6 zeigt das Ergebnis.

Tabelle 6

| Zugesetzte Staphylo-kokken-Konzentrationen (mg Staphylokokken/ml Testansatz) | $OD_{405nm}$ | Stimulierungsfaktor |
|---|---|---|
| 0 | 0,151 | 1,00 |
| $10^{-4}$ | 0,151 | 1,00 |
| $10^{-3}$ | 0,151 | 1,00 |
| $10^{-2}$ | 0,180 | 1,19 |
| $10^{-1}$ | 0,204 | 1,35 |
| 1 | 1,018 | 6,74 |
| 10 | 0,394 | 2,61 |

**Ansprüche**

1. Verfahren zur Bestimmung eines Plasminogenaktivators (PA) aus der Gruppe Urokinase (UK) und Gewebe-Plasminogenaktivator (t-PA) in Körperflüssigkeiten, dadurch gekennzeichnet, daß der Plasminogenaktivator mit Plasminogen und einem Polyschwefelsäureester eines Saccharids oder einem sulfatierten Zucker und gegebenenfalls mit Fibrin oder Fibrin-Spaltprodukten inkubiert und die Umsetzung von Plasminogen zu Plasmin gegebenenfalls unter Verwendung eines chromogenen Substrats bestimmt wird.

2. Verfahren nach Anspruch 1, dadurch gekennzeichnet, daß als Polyschwefelsäureester eines Saccharids oder als sulfatierter Zucker Zellmembranbestandteile verwendet werden.

3. Verfahren nach Anspruch 1, dadurch gekennzeichnet, daß der Plasminogenaktivator t-PA ist.

4. Verfahren nach Anspruch 1, dadurch gekennzeichnet, daß der Polyschwefelsäureester eines Saccharids Heparin ist.

5. Verfahren nach Anspruchen 1, dadurch gekennzeichnet, daß Heparin in einer Konzentration von $10^{-8}$ bis 1 mg/ ml Testansatz eingesetzt wird.

6. Verfahren nach Anspruch 1, dadurch gekennzeichnet, daß der Polyschwefelsäureester eines Saccharids oder sulfatierte Zucker Heparan-Sulfat, Dermatansulfat, Chondroitinsulfat, Pentosansulfat, Keratansulfat oder Mucopolysaccharidpolyschwefelsäureester in einer Konzentration $10^{-8}$ bis 1 mg/ml im Testansatz ist.

7. Verfahren nach Anspruch 1, dadurch gekennzeichnet, daß als Polyschwefelsäureester eines Saccharids oder als sulfatierter Zucker abgetötete Staphylokokken in einer Konzentration von $10^{-5}$ bis $10^{-2}$ g/ml im Testansatz eingesetzt werden.

8. Verfahren nach Anspruch 1, dadurch gekennzeichnet, daß als Polyschwefelsäureester eines Saccharids oder als sulfatierter Zucker Thrombozyten in einer Konzentration von 250 bis 250000 Zellen/ml im Testansatz eingesetzt werden.

9. Mittel zur Bestimmung eines Plasminogenaktivators mindestens enthaltend 0,01 bis 1 CTA/ml Plasminogen und $10^{-4}$ bis 1 mg/ml Heparin sowie gegebenenfalls 0,01 bis 0,1 ml/100 ml Polyoxyethylensorbitan-monooleat und gegebenenfalls Fibrinspaltprodukte.

10. Mittel zur Bestimmung eines Plasminogenaktivators mindestens enthaltend 0,01 bis 1 CTA/ml Plasminogen und $2,5 \times 10^3$ bis $2,5 \times 10^5$ Thrombozyten/ml sowie gegebenenfalls 0,01 bis 0,1 ml/100 ml Polyoxyethylensorbitan-monooleat und gegebenenfalls Fibrinspaltprodukte.

11. Mittel zur Bestimmung eines Plasminogenaktivators mindestens enthaltend 0,01 bis 1 CTA/ml Plasminogen und $10^{-1}$ bis 10 mg Staphylokokken/ml sowie gegebenenfalls 0,01 bis 0,1/100 ml Polyoxyethylensorbitan-monooleat und gegebenenfalls Fibrinspaltprodukte.

**Claims**

1. A method for the determination of a plasminogen activator (PA) from the group comprising urokinase (UK) and tissue plasminogen activator (t-PA) in body fluids, which comprises the plasminogen activator being incubated with plasminogen and with a polysulfuric ester of a saccharide or with a sulfated sugar and, where appropriate, with fibrin or fibrin degradation products, and the conversion of plasminogen into plasmin being determined, where appropriate, using a chromogenic substrate.

2. The method as claimed in claim 1, wherein cell membrane constituents are used as the polysulfuric ester of a saccharide or as the sulfated sugar.

3. The method as claimed in claim 1, wherein the plasminogen activator is t-PA.

4. The method as claimed in claim 1, wherein the polysulfuric ester of a saccharide is heparin.

5. The method as claimed in claim 1, wherein heparin is used in a concentration of $10^{-8}$ to 1 mg/ml of assay mixture.

6. The method as claimed in claimed in claim 1, wherein the polysulfuric ester of a saccharide or the sulfated sugar is heparan sulfate, dermatan sulfate, chondroitin sulfate, pentosan sulfate, keratan sulfate or mucopolysaccharide polysulfate, in a concentration of $10^{-8}$ to 1 mg/ml in the assay mixture.

7. The method as claimed in claim 1, wherein killed Staphylococci are used, in a concentration of $10^{-5}$ to $10^{-2}$ g/ml in the assay mixture, as the polysulfuric ester of a saccharide or as the sulfated sugar.

8. The method as claimed in claim 1, wherein platelets are used, in a concentration of 250 to 250,000 cells/ml in the assay mixture, as the polysulfuric ester of a saccharide or as the sulfated sugar.

9. An agent for the determination of a plasminogen activator at least containing 0.01 to CTA/ml plasminogen and $10^{-4}$ to 1 mg/ml heparin and, where appropriate, 0.01 to 0.1 ml/100ml polyoxyethylene sorbitan monooleate and, where appropriate, fibrin degradation products.

10. An agent for the determination of a plasminogen activator at least containing 0.01 to 1 CTA/ml plasminogen and $2.5 \times 10^3$ to $2.5 \times 10^5$ platelets/ml and, where appropriate, 0.01 to 0.1 ml/100 ml polyoxyethylene sorbitan monooleate and, where appropriate, fibrin degradation products.

11. An agent for the determination of a plasminogen activator at least containing 0.01 to 1 CTA/ml plasminogen and $10^{-1}$ to 10 mg of Staphylococci/ml and, where appropriate, 0.01 to 0.1 ml/100 ml polyoxyethylene sorbitan monoleate and, where appropriate, fibrin degradation products.


**Revendications**

1. Procédé de dosage d'un activateur de plasminogène (PA) du groupe de l'urokinase (UK) et de l'activateur de plasminogène tissulaire (t-PA) dans les fluides corporels, caractérisé en ce que l'on fait incuber ce PA avec du plasminogène et un poly(ester sulfurique) d'un saccharide ou un sucre sulfaté, et éventuellement avec de la fibrine ou des produits de dégradation de la fibrine, et en ce que l'on évalue la conversion du plasminogène en plasmine, éventuellement en utilisant un substrat chromogène.

2. Procédé selon la revendication 1, caractérisé en ce que l'on utilise, comme poly(ester sulfurique) d'un saccharide ou comme sucre sulfaté, des constituants de la membrane cellulaire.

3. Procédé selon la revendication 1, caractérisé en ce que l'activateur de plasminogène est le t-PA.

4. Procédé selon la revendication 1, caractérisé en ce que le poly(ester sulfurique) d'un saccharide est l'héparine.

5. Procédé selon la revendication 1, caractérisé en ce que l'héparine est mise en oeuvre à une concentration de $10^{-8}$ à 1 mg/ml de la prise d'essai.

6. Procédé selon la revendication 1, caractérisé en ce que le poly(ester sulfurique) de saccharide ou sucre sulfaté est le sulfate d'héparane, sulfate de dermatane, sulfate de chondroïtine, sulfate de pentosane, sulfate de kératane ou (polysulfate de mucopolysaccharide), à une concentration de $10^{-8}$ à 1 mg/ml dans la prise d'essai.

7. Procédé selon la revendication 1, caractérisé en ce que l'on utilise, comme poly(ester sulfurique) d'un saccharide ou comme sucre sulfaté, des staphylocoques tués, à une concentration de $10^{-5}$ à $10^{-2}$ g/ml dans la prise d'essai.

8. Procédé selon la revendication 1, caractérisé en ce que l'on utilise, comme poly(ester sulfurique) d'un

9

saccharide ou comme sucre sulfaté, des thrombocytes, à une concentration de 250 a 250000 cellules/ml dans la prise d'essai.

9. Moyen de dosage d'un activateur de plasminogène renfermant au moins de 0,01 à 1 CTA/ml de plasminogène et de $10^{-4}$ à 1 mg/ml d'héparine ainsi qu'éventuellement de 0,01 à 0,1 ml/100 ml de monooléate de polyoxyéthylènesorbitan et éventuellement des produits de dégradation de la fibrine.

10. Moyen de dosage d'un activateur de plasminogène renfermant au moins de 0,01 à 1 CTA/ml de plasminogène et de $2,5.10^3$ à $2,5.10^5$ thrombocytes/ml ainsi qu'éventuellement de 0,01 à 0,1 ml/100 ml de monooléate de polyoxyéthylènesorbitan et éventuellement des produits de dégradation de la fibrine.

11. Moyen de dosage d'un activateur de plasminogène renfermant au moins de 0,01 à 1 CTA/ml de plasminogène et de $10^{-1}$ à 10 mg de staphylocoques/ml ainsi qu'éventuellement de 0,01 à 0,1 ml/100 ml de monooléate de polyoxyéthylènesorbitan et éventuellement des produits de dégradation de la fibrine.